# EUROPEAN PATENT APPLICATION

(11) **EP 4 610 222 A1**
(43) Date of publication of application: **03.09.2025**
(21) Application number: 23882881.8
(22) Date of filing: 05.09.2023
(51) Int. Cl.: C01B 11/02, A01K 51/00, A61L 2/20, A61L 101/08, A61L 101/36, A61L 101/50

(54) **SUSTAINED-RELEASE CHLORINE DIOXIDE GAS GENERATION KIT AND METHOD**

(30) Priority: 28.10.2022 KR 20220141844; 25.04.2023 KR 20230054371
(71) Applicant: ABC Medical Co., Ltd., Anyang-si, Gyeonggi-do, 14118 (KR); Tahk, Joon Bae, Anyang-si, Gyeonggi-do, 14118 (KR)
(72) Inventor: CHOI, Jong Keun, Suwon-si Gyeonggi-do 16582 (KR); TAHK, Joon Bae, Anyang-si, Gyeonggi-do, 14118 (KR)
(74) Representative: J A Kemp LLP
(86) International application number: PCT/KR2023/013214
(87) International publication number: WO 2024/090774

(57) **Abstract**

The present invention relates to a generation method for generating chlorine dioxide gas in a sustained manner over a long period of time without generating dangerously high concentrations of gas. When using the sustained-release chlorine dioxide gas generation method of the present invention, chlorine dioxide gas can be produced at a low concentration without damaging bees for infection control of bee colonies, thereby achieving the purpose of long-term disinfection until a colony is replaced.

## Description

### Technical Field

This application claims priority and the benefit of Korean Patent Application No. 10-2022-0141844 filed in the Korean Intellectual Property Office on 28 October 2022 and Korean Patent Application No. 10-2023-0054371 filed in the Korean Intellectual Property Office on 25 April 2023, the disclosures of which are incorporated herein by reference.

The disclosure relates to a chlorine dioxide generation kit and method for generating chlorine dioxide gas in a sustained-release manner for several months.

### Background Art

It has been well known that the irradiation of ultraviolet light on aqueous solutions or gels containing sodium chlorite generates chlorine dioxide in the conventional art (Patent Document 1: Japanese Patent Publication No. 2005-224386 published on 25 August 2005). However, the irradiation of ultraviolet light on an aqueous solution containing sodium chlorite dissolved in pure water results in a too slow reaction and a small generation of sodium chlorite, thus failing to generate chlorine dioxide at a concentration that is enough for disinfection or sterilization. It has also been known that the contact of sodium chlorite with an acid such as citric acid generates chlorine dioxide (Patent Document 2: Korean Patent No. 10-1807966 registered on 5 December 2017), but the contact between sodium chlorite and citric acid cannot be stopped once the reaction initiates, and the use of sulfuric acid or chloric acid instead of citric acid is dangerous, and causes an excessive reaction, posing a risk of an explosion. Therefore, there has been a need for a method for stably producing chlorine dioxide gas at a low concentration.

As is well known, bees play an important role in plant pollination and thus are very essential for the maintenance of the ecosystem. However, bees are highly vulnerable to diseases caused by various causing pathogens, such as fungi, bacteria, and viruses, and chemicals, such as control pesticides and insecticides, for the purpose of improving crop productivity, often act on bees instead, causing significant damage to a colony of bees. Especially, bee colony collapse disorder (CCD) has recently occurred in the United States, Canada, Europe, South America, Asia, and the like, and the exact cause of this colony collapse disorder has not been revealed, but is known to result from a complex action of various diseases, pests, bacteria, and viral pathogens prevalent in bees. Therefore, there is a need to develop a disinfectant with excellent disinfecting effects that is harmless to bees themselves and does not remain in the honey.

Korean Patent No. 10-1315260 (Patent Document 3, registered on 30 September 2013) discloses a disinfection device for the prevention and treatment of an infectious disease, such as sacbrood, by using chlorine dioxide gas. Chlorine dioxide is hard to store or transport due to its explosion at a concentration above 10%, and thus the patent discloses that stabilized chlorine dioxide, obtained by a reaction with an alkaline salt, reacts with ultraviolet light, leading to a safe generation of chlorine dioxide gas and excellent disinfecting effects. However, said device failed to be commercialized since the device was difficult to manage due to the inclusion of an electrical apparatus.

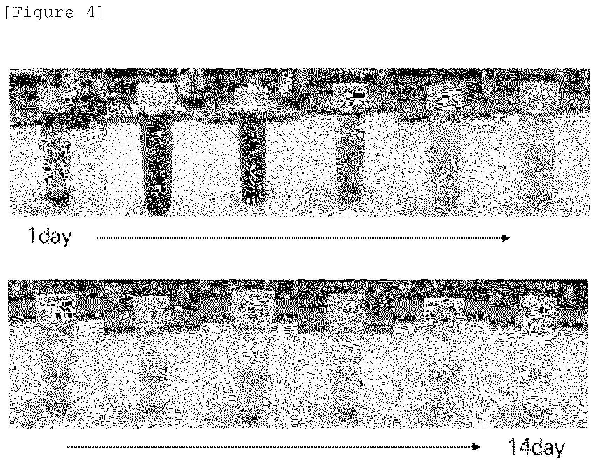

Moreover, the addition of citric acid to a hypochlorous acid solution in the conventional art generates chlorine dioxide very explosively, but while the gas generated by dissolving chlorine dioxide in the solution is gradually released, the amount of gas generated decreases, and thus the gas generation ended within up to about 2 weeks (see FIG. 4). Therefore, there is a need to develop a method and kit capable of stably generating chloride dioxide gas at a low concentration for a long time, even without including an electrical apparatus.

### Disclosure of Invention

### Technical Problem

As described above, there is a need to develop a safe and long-term easy-to-use disinfection method for preventing or treating infectious diseases in bees. Especially for bees, unlike other livestock, quarantining only the infected bees is impossible when an infectious disease arises, and all causative pathogens cannot be completely eradicated from infected bees. Therefore, for the infection management in the bee colony, it is necessary to create an environment that allows the disinfection of the entire bee colony and sustain this environment for at least 60 days, the average lifespan of a bee, thereby inducing the replacement of the colony with an uninfected bee generation.

Therefore, the present inventors have made intensive research efforts to develop a method and apparatus capable of producing chlorine dioxide gas safely and consistently for a long time while ensuring ease of use for the bee colony management. As a result, the present inventors identified that the addition of an organic substance containing a hydroxy (-OH) group and an organic acid to a gel composition containing sodium chlorite generates chlorine dioxide at a low concentration for a long time, and thus completed the disclosure.

Accordingly, an aspect of the disclosure is to provide a sustained-release chlorine dioxide gas generating kit including (a) a chlorite, (b) a sugar, (c) a gelling agent or thickener, and (d) an acid.

Another aspect of the disclosure is to provide a method for delayed generation of chlorine dioxide gas, the method including adding, into a container containing an agent including (a) a chlorite, (b) a sugar, and a powder including an acid.

### Solution to Problem

In accordance with an aspect of the disclosure, there is provided a chlorine dioxide gas generating kit including:
(a) a chlorite, (b) a sugar, (c) a gelling agent or thickener, and (d) an acid.

The chlorite of the disclosure reacts with a sugar to consistently generate chlorine dioxide gas for a long time. The types of the sugar may be listed in Table 1 below, but are not limited thereto. However, the reaction of the chlorite (a) and the sugar (b) initiates too slowly, making it impossible to specify the reaction initiation time. Moreover, when the chlorite and the sugar are agitated, the reaction rate may become uncontrollable, causing a risk of explosion.

Therefore, the acid (d) is further included in the chlorine dioxide gas generating kit including the chlorite (a), the sugar (b), the gelling agent or thickener (c), thereby initiating a fast reaction with the chlorite. As a result, chlorine dioxide gas was promptly generated by a reaction of the acid and the chlorite, and the chlorine dioxide gas again accelerated a reaction of the chlorite and the sugar. That is, among the components of the kit, the acid can promptly react with the chlorite to control the time of chlorine dioxide gas generation reaction, and the gelling agent or thickener can delay the generation of chlorine dioxide to generate chlorine dioxide gas in a sustained-release manner for a long time.

In an embodiment of the disclosure, the chlorite is at least one selected from the group consisting of sodium chlorite, potassium chlorite, lithium chlorite, calcium chlorite, magnesium chlorite, and barium chlorite, but is not limited thereto.

In an embodiment of the disclosure, the chlorite may be contained in a weight of 1 to 15%, 1 to 10%, 1 to 9%, 1 to 8%, 1 to 7%, 1 to 6%, 1 to 5%, 2 to 15%, 2 to 10%, 2 to 9%, 2 to 8%, 2 to 7%, 2 to 6%, 2 to 5%, 3 to 15%, 3 to 10%, 3 to 9%, 3 to 8%, 3 to 7%, 3 to 6%, 3 to 5%, 4 to 15%, 4 to 10%, 4 to 9%, 4 to 8%, 4 to 7%, 4 to 6%, 4 to 5%, 5 to 15%, 5 to 10%, 5 to 9%, 5 to 8%, 5 to 7%, or 5 to 6% relative to the total weight of the components of the kit including (a) to (d) excluding the container, but is not limited thereto.

In an embodiment of the disclosure, the chlorite is a precursor of chlorine dioxide gas.

An agent including the chlorite of the disclosure may be provided in the form of an aqueous solution or gel, but the gel form is preferable for controlling the chlorine dioxide gas generation reaction rate.

In an embodiment of the disclosure, the sugar may be a monosaccharide, a disaccharide, a sugar alcohol, or a combination thereof. Specifically, the kit of the disclosure may include a monosaccharide and a disaccharide, or may include a monosaccharide, a disaccharide, and a sugar alcohol.

In an embodiment of the disclosure, the monosaccharide may be glucose, fructose, galactose, or a combination thereof.

In an embodiment of the disclosure, the monosaccharide may be contained in a weight of 0.05 to 5%, 0.05 to 4%, 0.05 to 3.5%, 0.05 to 3%, 0.05 to 2.5%, 0.05 to 2%, 0.05 to 1.5%, 0.05 to 1%, 0.05 to 0.9%, 0.05 to 0.8%, 0.05 to 0.7%, 0.05 to 0.6%, 0.05 to 0.5%, 0.05 to 0.4%, 0.05 to 0.35%, 0.05 to 0.3%, 0.05 to 0.25%, 0.05 to 0.2%, 0.1 to 5%, 0.1 to 4%, 0.1 to 3.5%, 0.1 to 3%, 0.1 to 2.5%, 0.1 to 2%, 0.1 to 1.5%, 0.1 to 1%, 0.1 to 0.9%, 0.1 to 0.8%, 0.1 to 0.7%, 0.1 to 0.6%, 0.1 to 0.5%, 0.1 to 0.4%, 0.1 to 0.35%, 0.1 to 0.3%, 0.1 to 0.25%, or 0.1 to 0.2% relative to the total weight of the components of the kit including (a) to (d) excluding the container, but is not limited thereto.

In an embodiment of the disclosure, the disaccharide may be sucrose, maltose, trehalose, lactose, or a combination thereof.

In an embodiment of the disclosure, the disaccharide may be contained in a weight of 1 to 10%, 1 to 9%, 1 to 8%, 1 to 7%, 1 to 6%, 1 to 5%, 1 to 4%, 2 to 10%, 2 to 9%, 2 to 8%, 2 to 7%, 2 to 6%, 2 to 5%, 2 to 4%, 3 to 10%, 3 to 9%, 3 to 8%, 3 to 7%, 3 to 6%, 3 to 5%, or 3 to 4% relative to the total weight of the components of the kit including (a) to (d) excluding the container, but is not limited thereto.

In an embodiment of the disclosure, the disaccharide is a main reactant that drives a main reaction after the reaction between the chlorite and the monosaccharide at the initial stage.

In an embodiment of the disclosure, the sugar alcohol may be ethylene glycol, glycerol, erythritol, sorbitol, mannitol, xylitol, inositol, or a combination thereof.

In an embodiment of the disclosure, the sugar alcohol may be contained in a weight of 1 to 10%, 1 to 9%, 1 to 8%, 1 to 7%, 1 to 6%, 1 to 5%, 1 to 4%, 2 to 10%, 2 to 9%, 2 to 8%, 2 to 7%, 2 to 6%, 2 to 5%, 2 to 4%, 3 to 10%, 3 to 9%, 3 to 8%, 3 to 7%, 3 to 6%, 3 to 5%, or 3 to 4% relative to the total weight of the components of the kit including (a) to (d) excluding the container, but is not limited thereto.

In an embodiment of the disclosure, the sugar alcohol is a main reactant that drives a main reaction after the reaction between the chlorite and the monosaccharide at the initial stage.

In an embodiment of the disclosure, examples of the gelling agent or thickener may include agar, Konjac powder, carrageenan, pectin, gelatin, starch, locust bean gum, tara gum, guar gum, gellan gum, xanthan gum, tamarind gum, arabic gum, cellulose gum, sodium caseinate, sodium alginate, dextran, dextrin, carboxymethyl cellulose, or a combination thereof.

In an embodiment of the disclosure, the gelling agent or thickener may be contained in a weight of 1 to 10%, 1 to 9%, 1 to 8%, 1 to 7%, 1 to 6%, 1 to 5.5%, 1 to 5%, 1 to 4%, 1 to 3%, 1 to 2%, 1 to 1.5%, 1.1 to 10%, 1.1 to 9%, 1.1 to 8%, 1.1 to 7%, 1.1 to 6%, 1.1 to 5.5%, 1.1 to 5%, 1.1 to 4%, 1.1 to 3%, 1.1 to 2%, 1.1 to 1.5%, 1.2 to 10%, 1.2 to 9%, 1.2 to 8%, 1.2 to 7%, 1.2 to 6%, 1.2 to 5.5%, 1.2 to 5%, 1.2 to 4%, 1.2 to 3%, 1.2 to 2%, 1.2 to 1.5%, 1.3 to 10%, 1.3 to 9%, 1.3 to 8%, 1.3 to 7%, 1.3 to 6%, 1.3 to 5.5%, 1.3 to 5%, 1.3 to 4%, 1.3 to 3%, 1.3 to 2%, 1.3 to 1.5%, 1.5 to 10%, 1.5 to 9%, 1.5 to 8%, 1.5 to 7%, 1.5 to 6%, 1.5 to 5.5%, 1.5 to 5%, 1.5 to 4%, 1.5 to 3%, 1.5 to 2%, 2 to 10%, 2 to 9%, 2 to 8%, 2 to 7%, 2 to 6%, 2 to 5.5%, 2 to 5%, 2 to 4%, 2 to 3%, 3 to 10%, 3 to 9%, 3 to 8%, 3 to 7%, 3 to 6%, 3 to 5.5%, 3 to 5%, 3 to 4%, 4 to 10%, 4 to 9%, 4 to 8%, 4 to 7%, 4 to 6%, 4 to 5.5%, 4 to 5%, 5.5 to 10%, 5.5 to 9%, 5.5 to 8%, 5.5 to 7%, 5.5 to 6%, 5 to 10%, 5 to 9%, 5 to 8%, 5 to 7%, 5 to 6%, or 5 to 5.5% relative to the total weight of the components of the kit including (a) to (d) excluding the container, but is not limited thereto.

In an embodiment of the disclosure, the gelling agent or thickener is a delay reactant that delays the reaction with the chlorite.

In an embodiment of the disclosure, the kit includes a gelation accelerator.

In a specific embodiment of the disclosure, the gelation accelerator is magnesium chloride, potassium chloride, calcium chloride, sodium chloride, or a combination thereof, but is not limited thereto.

In an embodiment of the disclosure, the acid is an organic acid, an inorganic acid, or a mixture thereof.

In an embodiment of the disclosure, examples of the organic acid include lactic acid, acetic acid, formic acid, citric acid, oxalic acid, ascorbic acid, glucuronic acid, maleic acid, succinic acid, benzoic acid, tartaric acid, fumaric acid, propionic acid, glutamic acid, aspartic acid, butyric acid, or a combination thereof, but is not limited thereto.

In a specific embodiment of the disclosure, examples of the inorganic acid include hydrochloric acid, phosphoric acid, sulfuric acid, nitric acid, or a combination thereof, but is not limited thereto.

In an embodiment of the disclosure, the acid may be contained in a weight of 0.001 to 2%, 0.001 to 1.5%, 0.001 to 1%, 0.001 to 0.5%, 0.001 to 0.4%, 0.001 to 0.3%, 0.001 to 0.2%, 0.001 to 0.1%, 0.005 to 2%, 0.005 to 1.5%, 0.005 to 1%, 0.005 to 0.5%, 0.005 to 0.4%, 0.005 to 0.3%, 0.005 to 0.2%, 0.005 to 0.1%, 0.01 to 2%, 0.01 to 1.5%, 0.01 to 1%, 0.01 to 0.5%, 0.01 to 0.4%, 0.01 to 0.3%, 0.01 to 0.2%, 0.01 to 0.1%, 0.05 to 2%, 0.05 to 1.5%, 0.05 to 1%, 0.05 to 0.5%, 0.05 to 0.4%, 0.05 to 0.3%, 0.05 to 0.2%, or 0.05 to 0.1% relative to the total weight of the components of the kit including (a) to (d) excluding the container, but is not limited thereto.

In an embodiment of the disclosure, the chlorine dioxide gas generating kit of the disclosure may be composed of: a first agent including (a) a chlorite; a second agent including (b) a sugar and (c) a gelling agent or thickener; and a third agent including (d) an acid, but is not limited thereto.

In another embodiment of the disclosure, the chlorine dioxide gas generating kit may be composed of: a first agent including (a) a chlorite and (c) a gelling agent or thickener; and a second agent including (b) a sugar, (c) a gelling agent or thickener, and (d) an acid, but is not limited thereto.

In an embodiment, when the first agent contains the chlorite (a) and the gelling agent or thickener (c), the gelling agent or thickener serves to stabilize the chlorite.

In an embodiment, when the second agent contains the sugar (b), the gelling agent or thickener (c), and the acid (d), the gelling agent or thickener serves to delay the reaction where the acid reacts with the chlorite to generate chlorine dioxide.

In a specific embodiment of the disclosure, the chloride dioxide gas generating kit may include (a) a chlorite, (b) glucose and sucrose, (c) agar, starch, and xanthan gum, and (d) ascorbic acid.

In an embodiment of the disclosure, the kit is used for the disinfection, deodorization, or pesticide removal of an article, but is not limited thereto.

The agent including the chlorite of the disclosure may be provided in the form of being contained in a container.

In an embodiment of the disclosure, the sugar may be provided in a powder form.

In an embodiment of the disclosure, the gelling agent or thickener may be provided in a powder or gel form.

In an embodiment of the disclosure, the acid may be provided in a powder or liquid form.

As validated in the examples of the disclosure, the contact between the component preparations of the chlorine dioxide gas generating kit of the disclosure generates chlorine dioxide gas after several hours.

The concentration of chlorine dioxide gas may be maintained at a concentration of 0.5 to 1 ppm for the initial 1 to 3 days and maintained at a concentration of 0.03 to 0.05 ppm for 60 days or longer after 3 days.

It has been confirmed by the US Environmental Protection Agency that the chlorine dioxide of the disclosure is a safe sterilizing disinfectant due to the absence of carcinogenic trihalomethane generation in the purification treatment of drinking water. Additionally, the chlorine dioxide of the disclosure has been used to sterilize and disinfect anthrax. The chlorine dioxide is also a disinfectant that has received the A-1 grade, the safest standard among food additives, by the World Health Organization, and is approved by the U.S. Food and Drug Administration and the Ministry of Food and Drug Safety of South Korea as being usable for sterilization purposes in the washing of fruits, vegetables, food containers, and the like.

The chlorine dioxide of the disclosure, unlike hypochlorous acid (chlorine bleach) and chloramine which are other chlorine disinfectants, has high safety since it does not generate poisonous byproducts, such as trihalomethanes, haloacetonitriles, haloacetic acids, iodates, bromates, aldehydes, ketones, and benzene, during disinfection.

Additionally, the chlorine dioxide of the disclosure has excellent disinfection effects against various bacteria and viruses (Morino et al., Letters in Applied Microbiology 53, 628-634, 2011).

According to another aspect of the disclosure, a method for delayed generation of chlorine dioxide gas is provided including adding, into a container containing an agent including (a) a chlorite, (b) a sugar and a powder including an acid.

In an embodiment of the disclosure, the agent including a chlorite contained in the container may have a liquid or gel formulation.

In an embodiment of the disclosure, the agent including a chlorite contained in the container may contain purified water.

In an embodiment of the disclosure, (i) the agent contained in the container, (ii) the powder, or (iii) the agent contained in the container and the powder further include a gelling agent or a thickener.

In an embodiment of the disclosure, the container has an openable and closable lid, and the lid has a hole, through which chlorine dioxide gas generated in the container can escape from the container.

In an embodiment of the present disclosure, the hole may also have an openable and closable lid.

In an embodiment of the disclosure, the chlorine dioxide gas generation method is employed in the temperature conditions of 10 to 50°C.

In an embodiment of the disclosure, the replacement cycles of the agents used in the chlorine dioxide gas generating method are 2 to 3 months. The kit employed in the method of the disclosure is sustained for at least 60 days, the average lifespan of a bee, and thus can be used until the colony is replaced with a bee generation that is not infected with pathogens.

In an embodiment of the disclosure, the chlorine dioxide gas disinfects bacteria, viruses, or fungal pathogens in bees.

In an embodiment of the disclosure, the chlorine dioxide gas eliminates an herbicide. Examples of the herbicide include mancozeb, ethylenethiourea, tebuconazole, azoxystrobin, dimethomorph, methamidophos, or a combination thereof, but are not limited thereto.

### Advantageous Effects of Invention

The disclosure provides a kit and method for generating chlorine dioxide with a short storage time in a sustained-release manner over a long time while lowering the high gas generation in the early stage in the conventional art. The use of the chlorine dioxide gas generating kit and method enables the production of chlorine dioxide gas for a long time without harmfulness caused by high concentrations, so that chlorine dioxide gas can be stably produced until infected bees are replaced with an infected bee generation in the entire bee colony, thus achieving the sterilization or disinfection of spaces or objects.

### Brief Description of Drawings

FIG. 1 is an image showing that, into a container containing a first agent (sodium chlorite-including agar gel), a second agent (glucose and xanthan gum) in a powder form, and a third agent (ascorbic acid) in a powder form are added, in the kit of the disclosure.
FIG. 2 shows the results of measuring chlorine dioxide for 77 days from 3 hours after the addition of the first agent (sodium chlorite-including agar gel), the second agent (glucose and xanthan gum), and the third agent (ascorbic acid) of the disclosure.
FIG. 3 illustrates that a kit was manufactured for the purpose of disinfecting a 100-cubic-meter space by increasing the ratio of the first agent (sodium chlorite-including agar gel), the second agent (glucose and xanthan gum), and the third agent (ascorbic acid), and chlorine dioxide was generated at 1 ppm or more for 120 days.
FIG. 4 illustrates that the chlorine dioxide gas generation was implemented using citric acid according to the conventional art, and the gas was generated for up to 14 days at room temperature and then the gas generation was stopped
FIG. 5 shows a manufacture example of a sustained-release chlorine dioxide generation kit product of the disclosure.

### Mode for Carrying out the Invention

Hereinafter, the disclosure will be described in more detail through exemplary embodiments. These exemplary embodiments are provided only for the purpose of specifically illustrating the disclosure, and therefore, according to the purpose of the disclosure, it would be apparent to a person skilled in the art that these exemplary embodiments are not construed to limit the scope of the disclosure.

### Examples

Throughout the present specification, the "%" used to express the concentration of a specific material, unless otherwise particularly stated, refers to (wt/wt)% for solid/solid, (wt/vol)% for solid/liquid, and (vol/vol) % for liquid/liquid.

### Example 1: Chlorine dioxide generation test 1 - organic substance addition

To investigate the reactivity with and the risk of sodium chlorite depending on the type of organic substance, each of organic substances in Table 1 was added to a 23% aqueous solution of sodium chlorite to observe the reactivity at room temperature and refrigerated temperature. The types of organic substances and the reactivity results are shown in Table 1.

**TABLE 1**

| No. | Organic substance | Amount added | Chemical Formula | Reaction rate |
|---|---|---|---|---|
| 1 | Ethanol | 30%, 2 ml | C₂H₅OH | +++++ |
| 2 | Sodium glutamate | 0.3 g | C₅H₈NO₄Na | +++++ |
| 3 | Glucose | 0.3 g | C₆H₁₂O₆ | +++++ |
| 4 | Glycerol | 30%, 2 ml | C₃H₈O₃ | +++++ |
| 5 | Silica gel | 0.3 g | SiO₂nH₂O | +++++ |
| 6 | Sorbitol | 0.3 g | C₆H₁₄O₆ | +++ |
| 7 | Sucrose | 0.3 g | C₁₂H₂₂O₁₁ | +++ |
| 8 | Fructose | 0.3 g | C₆H₁₂O₆ | +++ |
| 9 | Oligosaccharide | 0.3 g | C₁₂H₂₂O₁₁ | +++ |
| 10 | Sodium saccharin | 0.3 g | C₇H₄O₃NS Na | +++ |
| 11 | Trehalose | 0.3 g | C₁₂H₂₂O₁₁ | +++ |
| 12 | Xanthan gum | 0.3 g | Unspecified | + |
| 13 | Carrageenan | 0.3 g | Unspecified | + |
| 14 | Starch | 0.3 g | (C₆H₁₀O₅) n | + |
| 15 | Dish soap | 30%, 2 ml | Surfactant, anion | + |
| 16 | Sodium alginate | 0.3 g | (C₆H₇NaO₆) n | + |
| 17 | Sodium carboxymethyl cellulose | 0.3 g | C₈H₁₆NaO₈ | + |
| 18 | Sodium carbonate | 0.3 g | NaHCO₃ | + |
| 19 | Agar | 0.3 g | Unspecified | - |
| 20 | Polypropylene | 0.3 g | (C₃H₆) n | - |
| 21 | Gellan gum | 0.3 g | Unspecified | - |
| 22 | Glucomannan | 0.3 g | Unspecified | - |
| 23 | Hydrogen peroxide | 30%, 2 ml | H₂O₂ | No reaction |
| 24 | Salt | 0.3 g | NaCl | No reaction |
| 25 | Calcium oxide | 0.3 g | CaO | No reaction |
| 26 | Sodium hydroxide | 0.3 g | NaOH | No reaction |
| 27 | Potassium hydroxide | 0.3 g | KOH | No reaction |
| 28 | Calcium hydroxide | 0.3 g | Ca(OH)₂ | No reaction |
| 29 | Canola oil | 2 ml | oil | No reaction |

The use of the organic substances with a high reaction rate (+++++) resulted in the initiation of chlorine dioxide gas generation relatively quickly within several hours to several days after the addition of the organic substances and also showed relative fast reactions. The use of the organic substances with an intermediate reaction rate (+++) resulted in the initiation of chlorine dioxide gas generation within several days to several tens of days after the addition of the organic substances and showed slower reactions than the organic substances with fast reaction rates. The reaction initiation time of the organic substances with a slow reaction rate (+) is hard to specify, and even when the reaction initiated, careful observation was needed to identify the tiny gas generation.

The reaction initiation time varied depending on the type of organic substance, and the reaction initiation times cannot be generally specified. However, when the chlorine dioxide gas generation reaction initiated, the use of the substances with a fast reaction rate resulted in a fast reaction, and the use of the substances with a slow reaction resulted in a slow reaction, extending the duration of gas generation.

### Example 2: Chlorine dioxide generation test 2 - organic substance and organic acid addition

The present inventors identified from Example 1 above that when the reaction with sodium chlorite occurred in an aqueous solution state, the addition of glucose with a fast reaction rate resulted in the initiation of the reaction on day 7 to day 20, and once the reaction was initiated, the reaction occurred consistently. However, the reaction occurred slowly compared with a conventional method for generating chlorine dioxide by addition of an organic acid to a chlorite.

In other words, the addition of an organic substance could generate chlorine dioxide gas at a concentration that is harmless, for a long time, but such a case is not practical since the reaction was initiated too slowly and the reaction initiation time could not be specified, and thus it was attempted to add a small amount of an organic acid to accelerate the reaction initiation.

As one example, a 35-ml gel solid was prepared using alkali-resistant agar including 0.3 g of sodium chlorite as a first agent, and 0.5 g of a glucose powder and 0.5 g of xanthan gum as a second agent and 0.02 g of ascorbic acid as a third agent were applied on the surface of the agar gel. As a result, the reaction slowly occurred on the surface of the agar gel, and the entire gel began to slowly release chlorine dioxide gas.

To measure the concentration of chlorine dioxide generated according to the chloride dioxide generation method, a 125-L chamber (width of 50 cm* length of 50 cm* height of 50 cm) was fabricated, and by using the above-described method, citric acid and a glucose powder were added to a sodium chlorite agar gel to generate chlorine dioxide gas, and the concentration of chlorine dioxide was measured after 1 hour, for 77 days at 7-day intervals.

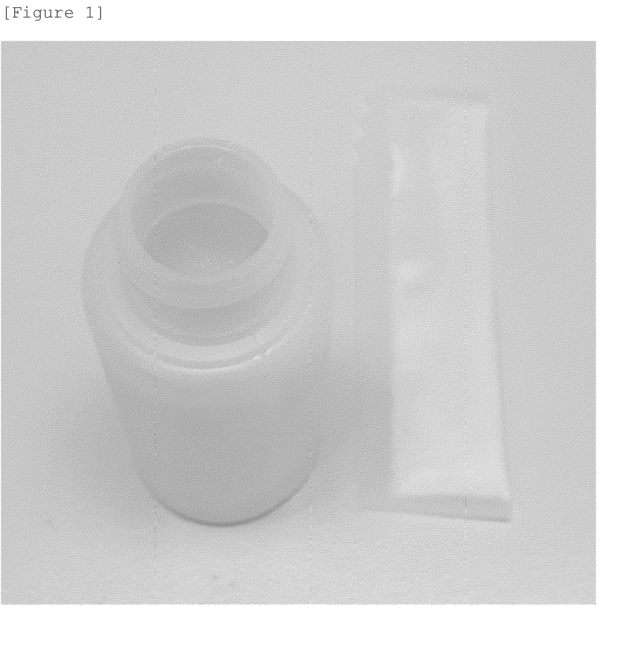

FIG. 1 is an image illustrating the addition of the second agent in a powder form and the third agent in a powder form to the container containing the first agent in the kit.

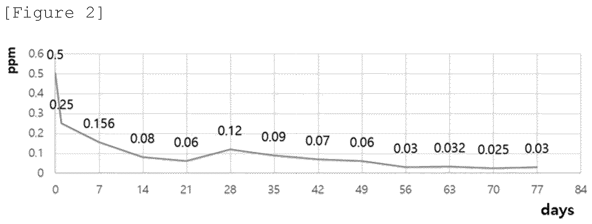

FIG. 2 shows the results of measuring the concentration of chlorine dioxide for 77 days.

As shown in FIG. 2, chlorine dioxide gas was generated several hours after the contact among the first, second, and third agents of the chlorine dioxide generation kit of the disclosure, and measured as a concentration of up to 0.5 ppm. The concentration was gradually reduced within several days and was measured as about 0.03 to about 0.05 ppm for up to 77 days.

In the infected bee colony, the time taken to grow from an egg to an adult is about 22 days, and the lifespan of the adult is about 35 days, so that a long-term disinfection of 60 days or longer is required to disinfect the entire bee colony. The above results confirmed that the generation of chlorine dioxide gas through the kit of the disclosure resulted in low concentrations of chlorine dioxide gas, which enables the generation of chlorine dioxide for a long time, about 60 days or longer, corresponding to the replacement cycle of a bee colony, without causing harm to the bees, thereby preventing the spread of infectious diseases throughout the bee colony and achieving sufficient disinfection effects.

### Example 3: Chlorine dioxide generation test 3

To investigate the amount of chlorine dioxide generated depending on the ratio of first, second, and third agents added, a chlorine dioxide generation test was conducted in the conditions shown in Table 2.

**TABLE 2**

| Water 100 ml + agar 1.5 g + NaClO2 10 ml (23% aqueous solution) | | | | | | |
|---|---|---|---|---|---|---|
| Classification | Control group | Examples 3-1 | Examples 3-2 | Examples 3-3 | Examples 3-4 | Examples 3-5 |
| Citric acid | - | - | 0.02 g | 0.02 g | 0.02 g | 0.02 g |
| Glucose | - | 1 g | - | - | 1 g | 0.5 g |
| Xanthan gum | - | - | - | 0.5 g | - | 0.5 g |
| Day 1 | 0 | 0 | 0.200 | 0.290 | 1.700 | 1.500 |
| Day 2 | 0 | 0 | 0.150 | 0.120 | 1.300 | 0.980 |
| Day 3 | 0 | 0 | 0.100 | 0.080 | 1.200 | 0.120 |
| Day 4 | 0 | 0 | 0.060 | 0.020 | 1.000 | 0.120 |
| Day 5 | 0 | 0 | 0.020 | 0.020 | 0.900 | 0.100 |
| Day 6 | 0 | 0.001 | 0.001 | 0.001 | 0.900 | 0.100 |
| Day 7 | 0 | 0 | 0 | 0 | 0.700 | 0.080 |
| Day 8 | 0 | 0 | 0 | 0 | 0.650 | 0.090 |
| Day 9 | 0 | 0 | 0 | 0 | 0.600 | 0.090 |
| Day 10 | 0 | 0 | 0 | 0 | 0.530 | 0.090 |
| Day 11 | 0 | 0 | 0 | 0 | 0.450 | 0.100 |
| Day 12 | 0 | 0 | 0 | 0 | 0.310 | 0.080 |
| Day 13 | 0 | 0 | 0 | 0 | 0.190 | 0.090 |
| Day 14 | 0 | 0.001 | 0 | 0 | 0.060 | 0.080 |
| Day 15 | 0 | 0.001 | 0 | 0 | 0.060 | 0.090 |
| Day 16 | 0 | 0.001 | 0 | 0 | 0.040 | 0.090 |
| Day 17 | 0 | 0.001 | 0 | 0 | 0.040 | 0.070 |
| Day 18 | 0 | 0.001 | 0 | 0 | 0.040 | 0.080 |
| Day 19 | 0 | 0.002 | 0 | 0 | 0.040 | 0.070 |
| Day 20 | 0 | 0.002 | 0 | 0 | 0.040 | 0.080 |
| Day 21 | 0 | 0.002 | 0 | 0 | 0.030 | 0.090 |
| Day 22 | 0 | 0.025 | 0 | 0 | 0.030 | 0.090 |
| Day 23 | 0 | 0.025 | 0 | 0 | 0.025 | 0.090 |
| Day 24 | 0 | 0.025 | 0 | 0 | 0.030 | 0.060 |
| Day 25 | 0 | 0.030 | 0 | 0 | 0.020 | 0.090 |
| Day 26 | 0 | 0.030 | 0 | 0 | 0.020 | 0.080 |
| Day 27 | 0 | 0.030 | 0 | 0 | 0.025 | 0.090 |
| Day 28 | 0 | 0.030 | 0 | 0 | 0.020 | 0.090 |
| Day 29 | 0 | 0.030 | 0 | 0 | 0.025 | 0.100 |
| Day 30 | 0 | 0.003 | 0 | 0 | 0.020 | 0.100 |

In Table 2 above, a gel was prepared by mixing water, agar, and a sodium chlorite solution, and citric acid, glucose, and xanthan gum prepared in a powder form were applied on the surface of the gel.

As shown in Table 2 above, in the control group, chlorine dioxide was not generated, and in Example 3-1 with addition of only glucose as an organic substance, chlorine dioxide was temporarily generated on the 6th day after the addition, and from the 14th day, chlorine dioxide generation gradually occurred.

In Example 3-2 and Example 3-3 with citric acid, chlorine dioxide generation was observed from the 1st day, but the chlorine dioxide generation occurred only up to day 6, and was no longer observed thereafter. In Example 3-3 with a further addition of xanthan gum, the amount of chlorine dioxide generated was more compared with the example with an addition of only citric acid.

In both Example 3-4 with citric acid and glucose and Example 3-5 with an addition of 0.5 g of glucose and 0.5 g of xanthan gum, the chlorine dioxide generation was observed from the 1st day and consistently observed for 30 days. However, in Example 3-4 with an addition of 1 g of only glucose, compared with Example 3-5 with an addition of 5 g each of glucose and xanthan gum, the amount of chloride dioxide generated was more in the early stage and relatively rapidly reduced. In Example 3-5 with an addition of 0.5 g each of glucose and xanthan gum, the amount of chloride dioxide generated in the early stage was small, but the amount of generation was maintained at a relatively higher concentration for a long time.

The above results indicated that an organic acid as the third agent and an organic substance as a second agent each react with sodium chlorite to generate chlorine dioxide gas. It can be therefore seen that the amount of chlorine dioxide generated varies depending on the amounts of an organic acid and an organic substance added.

It can also be seen that an organic acid such as citric acid has an effect of initiating a chlorine dioxide generation reaction, and an organic substance such as glucose, showing a fast reaction rate in Table 1, accelerates the reaction of glucose and sodium chlorite upon the reaction is initiated by the addition of the organic acid, thereby promptly generating chlorine dioxide gas.

Considering that the addition of an organic substance, such as xanthan gum, showing a relatively slow reaction rate, mixed with glucose generated chlorine dioxide gas at a concentration lower than the addition of only glucose, for a long time, it was identified that glucose was first involved in the reaction and xanthan gum was later involved in the reaction. Therefore, the chlorine dioxide generation kit and method of the disclosure can easily control the reaction rate and the use period of the kit by adjusting the mixing ratio of an organic substance showing a fast reaction and an organic substance showing a slow reaction.

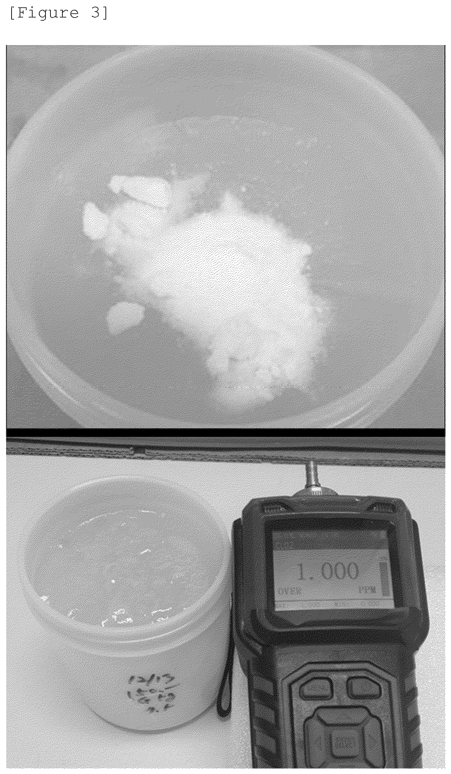

The present inventors manufactured a kit for the purpose of disinfecting a 100-cubic-meter space by increasing the amounts of the first, second, and third agents under the same ratio conditions as in Example 3-5. As shown in FIG. 3, chlorine dioxide gas was still generated at a concentration of 1 ppm on 12 April 2022, 4 months (120 days) after a chlorine dioxide gas generation kit manufactured on 13 December 2021.

FIG. 4 illustrates that when the chlorine dioxide gas was generated using citric acid and a sodium chlorite aqueous solution according to the conventional art, the gas was generated for up to 14 days at room temperature and the gas generation was stopped.

### Example 4: Manufacturing of sustained-release chloride dioxide gas generation kit

A sustained-release chlorine dioxide generation kit product was manufactured according to the compositions shown in Tables 3 and 4.

### <Composition of sustained-release chlorine dioxide generation kit product (BeeO₂)>

**TABLE 3**

| Composition of solid (85 ml) including sodium chlorite and gelling agent | | | |
|---|---|---|---|
| Component name | CAS No | Mixing proportion (%) | Use (function) |
| Purified water | 7732-18-5 | 81-82 | Dilution |
| Sodium chlorite | 7758-19-2 | 5.0-6.0 | Precursor |
| Agar | 9002-18-0 | 1.2-1.3 | Gelation |

**TABLE 4**

| Composition of powder additive (11 g) including sugar, gelling agent/thickener, and acid | | | |
|---|---|---|---|
| Ascorbic acid | 50-81-7 | 0.01-0.1 | Trigger |
| Glucose | 50-99-7 | 0.2-0.3 | Initial reactant |
| Starch | 9005-25-8 | 0.2-0.3 | Delay reactant |
| Sucrose | 57-50-1 | 5.0-6.0 | Main reactant |
| Xanthan gum | 11138-66-2 | 5.5-6.0 | Delay reactant |

The present inventors manufactured a kit by using the same compositions as in Tables 3 and 4, and chlorine dioxide gas was generated using the kit. As a result, although the kit having the same compositions as in Tables 3 and 4 had a chlorine dioxide gas generation duration of 3 months or more at room temperature, the chlorine dioxide gas generation duration was slightly reduced to 9-10 weeks since the internal temperature of the beehive was approximately 35°C, which was higher than room temperature.

Therefore, in order to generate the gas for 3 months or longer in an environment with a temperature reaching around 35°C, glucose was excluded from the powder additive and agar was added for reaction delay. As a result, a sustained-release chlorine dioxide generation kit product having the same compositions as in Tables 5 and 6 was manufactured, and chlorine dioxide gas was generated using the kit.

The kit having the same compositions as in Tables 5 and 6, compared with the kit having the same compositions as in Tables 3 and 4, showed an overall delayed reaction rate, and as a result, the chlorine dioxide gas generation was maintained for 3 months or longer even in a beehive with a high temperature.

**TABLE 5**

| Composition of solid (85 ml) including sodium chlorite and gelling agent | | | |
|---|---|---|---|
| Component name | CAS No | Mixing proportion (%) | Use (function) |
| Purified water | 7732-18-5 | 81-82 | Dilution |
| Sodium chlorite | 7758-19-2 | 5.0-6.0 | Precursor |
| Agar | 9002-18-0 | 1.2-1.3 | Gelation |

**TABLE 6**

| Composition of powder additive (11 g) including sugar, gelling agent/thickener, and acid | | | |
|---|---|---|---|
| Component name | CAS No | Mixing proportion (%) | Use (function) |
| Ascorbic acid | 50-81-7 | 0.1-0.3 | Trigger |
| Starch | 9005-25-8 | 2-3 | Delay reactant |
| Sucrose | 57-50-1 | 50-70 | Main reactant |
| Agar | 9002-18-0 | 15-25 | Delay reactant |
| Xanthan gum | 11138-66-2 | 15-25 | Delay reactant |

The product was manufactured as a sterilizer or deodorizer for enclosed spaces, such as shoe cabinets, at room temperature, or articles, such as empty beehives

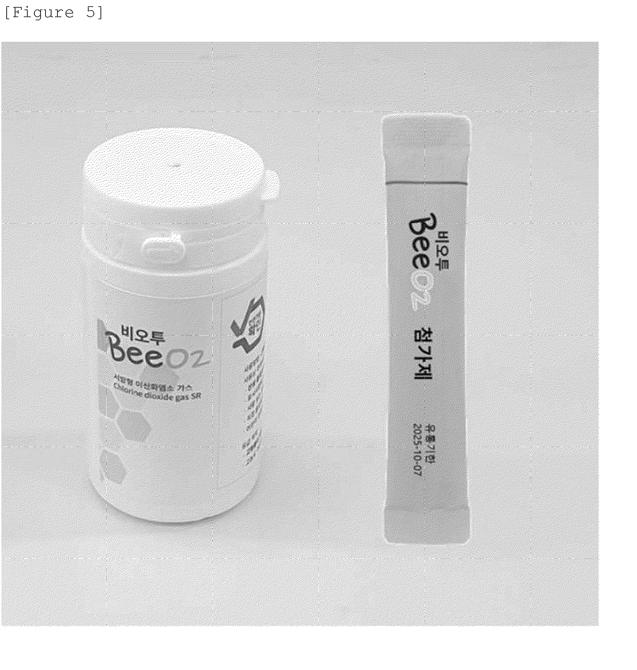

An image showing a product of the disclosure is shown in FIG. 5. For use, a sealed packaging is removed from a container containing a solid including purified water, sodium chlorite, and agar, and a powder additive, which is packaged in an attached stick pouch and includes ascorbic acid, glucose, starch, sucrose, and xanthan gum, is placed within the container, and then a lid is closed. As a result of the reaction, chlorine dioxide gas is generated.

The lid of the container has a small hole, so that chlorine dioxide gas slowly escapes to the outside of the container through the hole, even with the lid closed.

Although the present disclosure has been described in detail with reference to the specific features, it will be apparent to those skilled in the art that this description is only for a preferred embodiment and does not limit the scope of the present disclosure.

## Claims

1. A sustained-release chlorine dioxide gas generating kit comprising:
(a) a chlorite, (b) a sugar, (c) a gelling agent or thickener, and (d) an acid.

2. The kit of claim 1, wherein the chlorite is at least one selected from the group consisting of sodium chlorite, potassium chlorite, lithium chlorite, calcium chlorite, magnesium chlorite, and barium chlorite.

3. The kit of claim 1, wherein the sugar is a monosaccharide, a disaccharide, a sugar alcohol, or a combination thereof.

4. The kit of claim 1, wherein the monosaccharide is glucose, fructose, galactose, or a combination thereof.

5. The kit of claim 1, wherein the disaccharide is sucrose, maltose, trehalose, lactose, or a combination thereof.

6. The kit of claim 1, wherein the sugar alcohol is ethylene glycol, glycerol, erythritol, sorbitol, mannitol, xylitol, inositol, or a combination thereof.

7. The kit of claim 1, wherein the gelling agent or thickener includes agar, Konjac powder, carrageenan, pectin, gelatin, starch, locust bean gum, tara gum, guar gum, gellan gum, xanthan gum, tamarind gum, arabic gum, cellulose gum, sodium caseinate, sodium alginate, dextran, dextrin, carboxymethyl cellulose, or a combination thereof.

8. The kit of claim 1, wherein the acid is an organic acid, an inorganic acid, or a combination thereof.

9. The kit of claim 8, wherein the organic acid includes lactic acid, acetic acid, formic acid, citric acid, oxalic acid, ascorbic acid, glucuronic acid, maleic acid, succinic acid, benzoic acid, tartaric acid, fumaric acid, propionic acid, glutamic acid, aspartic acid, butyric acid, or a combination thereof.

10. The kit of claim 8, wherein the inorganic acid includes hydrochloric acid, phosphoric acid, sulfuric acid, nitric acid, or a combination thereof.

11. The kit of claim 1, wherein the kit is used for the disinfection, deodorization, or pesticide removal of an article.

12. A method for delayed generation of chlorine dioxide gas, the method comprising adding, into a container containing an agent including (a) a chlorite, (b) a sugar and a powder including acid.

13. The method of claim 12, wherein the agent contained in the container has a liquid or gel formulation.

14. The method of claim 12, wherein (i) the agent contained in the container, (ii) the powder, or (iii) the agent contained in the container and the powder include a gelling agent or a thickener.

15. The method of claim 12, wherein the container has an openable and closable lid, and the lid has a hole, through which chlorine dioxide gas generated in the container can escape from the container.
